# EUROPEAN PATENT SPECIFICATION

(11) **EP 0 420 911 B1**
(45) Date of publication and mention of the grant of the patent: **04.09.1996**
(21) Application number: 89907509.7
(22) Date of filing: 05.06.1989
(51) Int. Cl.: C12P 21/00, C07H 15/12

(54) **GENE THERAPY USING GENE FUSIONS FOR GENETIC OR ACQUIRED DISORDERS**
GENTHERAPIE UNTER VERWENDUNG VON GENSCHMELZEN FÜR GENETISCHE UND ERWORBENE STÖRUNGEN
THERAPIE GENETIQUE UTILISANT DES FUSIONS GENETIQUES POUR TROUBLES GENETIQUES OU ACQUIS

(30) Priority: 03.06.1988 US 202783
(43) Date of publication of application: 10.04.1991
(73) Proprietor: THE UNITED STATES OF AMERICA as represented by the Secretary United States Department of Commerce, Springfield, Virginia 22161 (US)
(72) Inventor: PASTAN, Ira, H., Potomac, MD 20854 (US); GOTTESMAN, Michael, M., Bethesda, MD 20817 (US); Germann, Ursula A., Brooklin, MA 02146 (US)
(74) Representative: Jump, Timothy John Simon
(86) International application number: US8902450
(87) International publication number: WO8912109

(56) References cited:
- EP-A- 0 095 361
- WO-A-90/04632
- WO-A-91/00361
- CHEMICAL ABSTRACTS, vol. 105, no. 1, 1986, Columbus, OH (US); D.A. WILLIAMS, p. 157, no. 1595w/
- PROCEEDINGS OF THE NATL. ACADEMY OF SCIENCES USA, vol. 84, May 1987, Washington, DC (US); K. UEDA et al., pp. 3004-3008/
- PROCEEDINGS OF THE NATL. ACADEMY OF SCIENCES USA, vol. 83, June 1986, Washington, DC (US); RONINSON et al., pp. 4538-4542/
- MOLECULAR & CELLULAR BIOLOGY, vol. 7, February 1987, McIVOR et al., pp. 838- 846/
- CHEMICAL ABSTRACTS, vol. 105, no. 19, 1986, Columbus, OH (US); P.W. KANTOFF, p. 192, no. 166144m/
- MOLECULAR & CELLULAR BIOLOGY, vol. 7, October 1987; LIM et al., pp. 3459-3465/
- FASEB JOURNAL, vol. 4, no. 5, March 1990, FASEB, Bethesda, MD (US); U.A. GERMANN et al., pp. 1501-1507/
- JOURNAL OF BIOLOGICAL CHEMISTRY, vol. 264, no. 13, 05 May 1989, Baltimore, US; U.A. GERMANN et al., pp. 7418-7424/

## Description

The present invention is related generally to the construction of fusion genes. More particularly, the present invention is related to the construction of a fusion gene comprising a coding sequence for a human MDR1 gene selectable marker linked by fusion to another coding sequence, the product of which is desired to be expressed in recipient cells.

Ueda et al. have described the isolation of a full-length cDNA for the human MDR1 gene and its expression in mouse NIH 3T3 and human KB cells, conferring a selectable multidrug resistance phenotype thereto (Proc. Natl. Acad. Sci. (1987), 84, 3004-3008).

Development in recombinant DNA technology and the need to treat genetic disorders has led to the concept of "gene therapy." To this end, methods have been developed for the introduction and expression of foreign genes into somatic cells. However, the expression of the delivered gene in the recipient cell in accordance with the prevalent methods is usually found to be either very low or quite variable.

Retrovirus-mediated transfer and expression of the ADA (adenosine deaminase) gene has been reported in a variety of human T and B lymphocyte cell lines, diploid human skin fibro-blasts, as well as in murine NIH 3T3 and lymphoid cells. More recently, a Maloney murine leukaemia virus-based recombinant retrovirus has been used to express ADA in murine haematopoietic stem cells (Lim, et al, 1987, Mol. Cell. Biol. 7, 3459). Such retroviral constructs, however, contained no dominant marker gene which would allow selection and thus efficient enrichment of ADA expressing cells.

It is, therefore, an object of the present invention to provide an efficient and reproducibly reliable method of introducing genes into animal or human cells to treat genetic or acquired disorders or defects caused by enzyme deficiency.

It is a further object of the present invention to provide a human MDR1 selectable marker-linked fusion gene for transfer and expression of a desired gene in human cells without introducing a non-human antigen.

It is another object of the present invention to provide gene therapy for the treatment of severe combined immunodeficiency caused by adenosine deaminase (ADA) deficiency.

Accordingly, in a first aspect of the present invention, there is provided a fusion gene capable of being expressed in vivo comprising a coding sequence for a selectable marker linked by fusion to a second coding sequence, resulting in a single open reading frame encoding a chimeric fusion protein, which it is desired to express in recipient cells, characterised in that the selectable marker is a human MDR1 gene.

In a second aspect of the present invention, there is provided the use of a fusion gene, according to the invention in its first aspect, for the manufacture of a medicament for use in a method for alleviating a condition resulting from a genetic or acquired disorder related to the second coding sequence, wherein the fusion gene is introduced into cells of a host and the second coding sequence is expressed in vivo.

In a third aspect of the invention, there is provided a method of determining the effect of altering the sequence of a fusion gene according to the invention in its first aspect, comprising the steps of altering the human MDR1 gene selectable marker coding sequence or the second coding sequence and determining the resultant effects of said alteration on the function of said fusion gene.

In a fourth aspect of the invention, there is provided a bifunctional chimeric protein encoded by a fusion gene according to the invention in its first aspect.

Preferred embodiments of the invention in any of its various aspects are as defined in the sub-claims.

These and other features and many of the attendant advantages of the invention will be better understood upon a reading of the following detailed description when considered in connection with the accompanying drawings wherein:
Figures 1 A,B,C schematically represent the construction of a retroviral expression vector pHaMDR1ADA which encodes a human P-glycoprotein-adenosine deaminase chimeric protein (it is noted that in Figures 1 A,B,C, MDRADA represents MDR1ADA);
Figure 2 shows Southern analysis of genomic DNAs of transfected KB cells. Genomic DNA was digested with EcoRI, separated on a 0.7% aqueous gel, transferred to nitrocellulose, and hybridized to a MDR 1-specific probe (pHDR5A). Panel A shows individually picked cell clones.
   Lane 1: KB-A, mock-transfected, grown at 6 ng/ml colchicine.
   Lane 2: KB-MDR1A, pHaMDR1 transfected, grown at 6 ng/ml colchicine.
   Lanes 3 and 4: KB-MDR1ADA-I, pHAMDR1A transfected, grown at 6 ng/ml (3) and 12 ng/ml (4) colchicine.
   Panel B shows pooled cell populations.
   Lanes 1 and 2: pHaMDR1 transfected, grown at 6 ng/ml (1) and 24 ng/ml (2) colchicine.
   Lanes 3 and 4: pHaMDR1ADA transfected, grown at 6 ng/ml (3) and 24 ng/ml (4) colchicine.
   Lane 5: non-transfected parental KB-3-1 cell line, colchicine-sensitive;

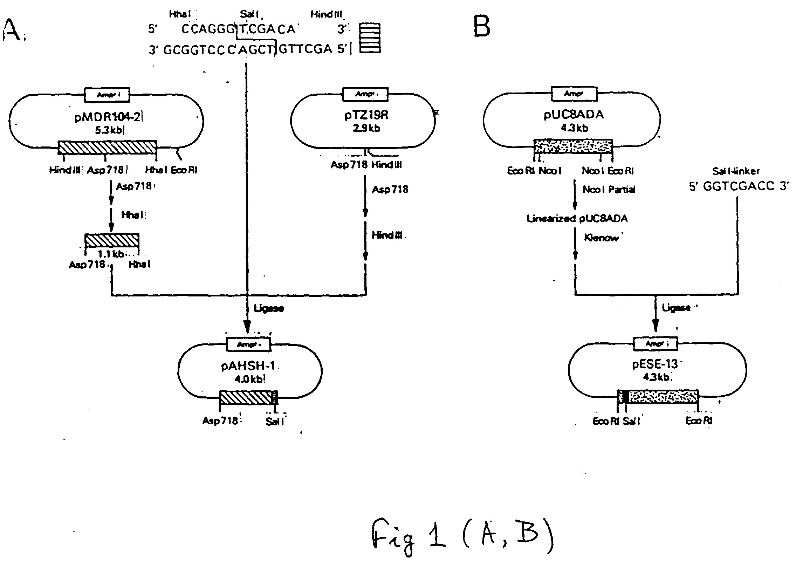

Figure 3 shows the results of immunoprecipitations of cell lysates. Cultures were labeled with ³⁵S-methionine for 16 hours. Cell lysates were immunoprecipitated using anti-P-glycoprotein antiserum (lanes 1 - 7) or preimmune serum (lanes 8 - 12) and protein A sepharose. Fluorograms of the resulting SDS-polyacrylamide gels are shown. Arrows indicate the 170 kD P-glycoprotein (MDR1 gene product) and the 210 kD P-glycoprotein-ADA fusion protein (MDR1ADA).
   Lane 1: drug-sensitive KB-3-1 control cell line, labelled and immunoprecipitated in parallel.
   Lane 2: vinblastine-selected KB-Vl control cell line.
   Lanes 3 and 8: pHaMDR1 transfected KB cell population grown at 24 ng/ml colchicine.
   Lanes 4, 5, 9, 10: pHaMDR1ADA transfected KB cell populations grown at 6 ng/ml (4 and 9) and 24 ng/ml (5 and 10) colchicine.
   Lanes 6, 7, 11, 12: pHaMDR1ADA transfected clone KB-MDR1ADA-I grown at 6 ng/ml (6 and 11) and 12 ng/ml (7 and 12) colchicine; and

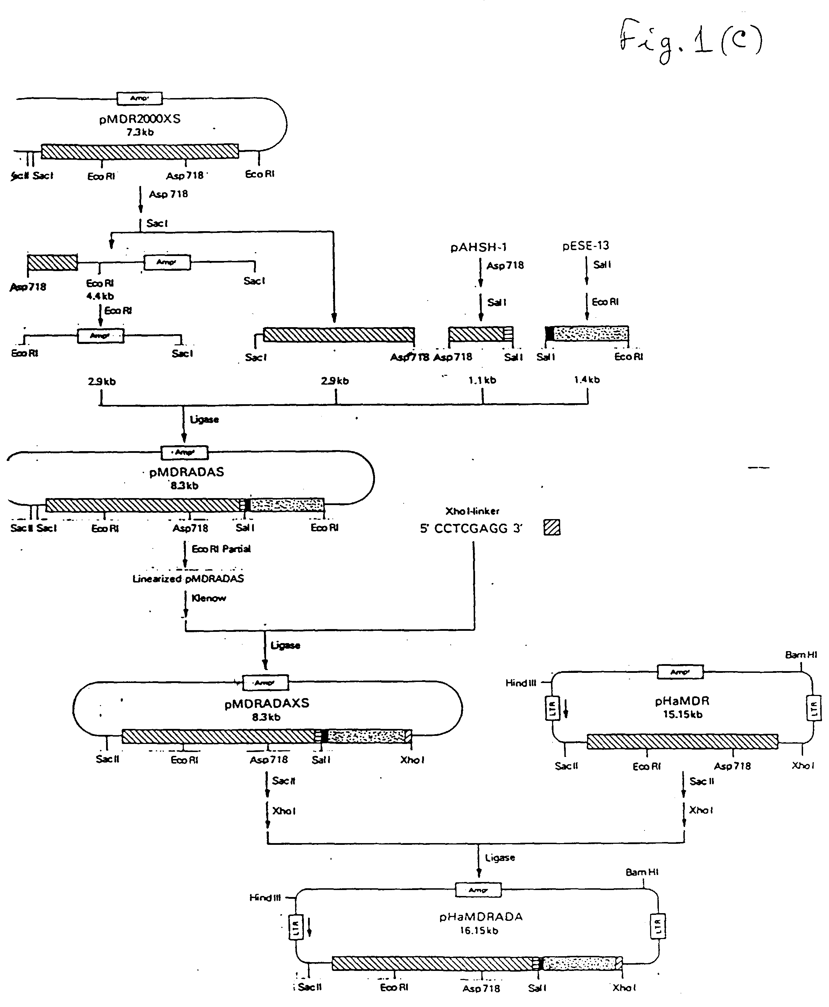

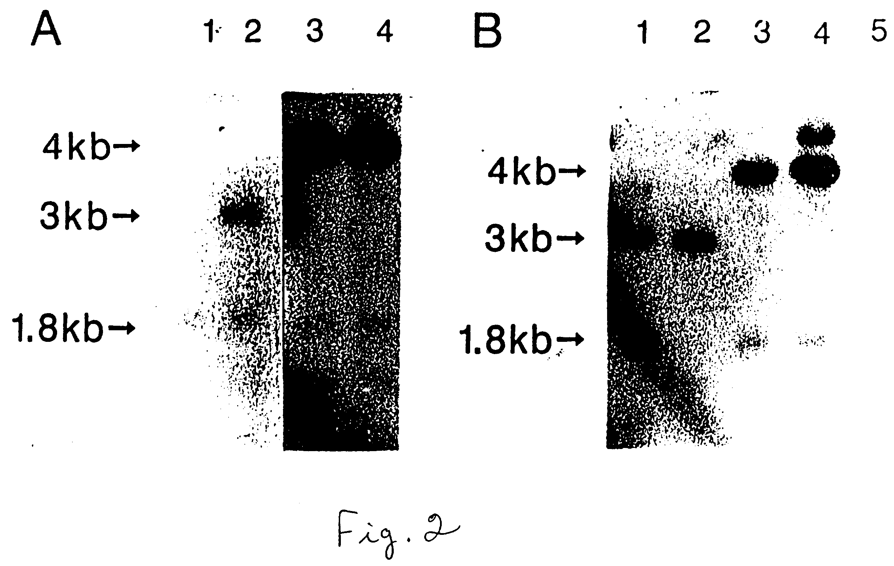

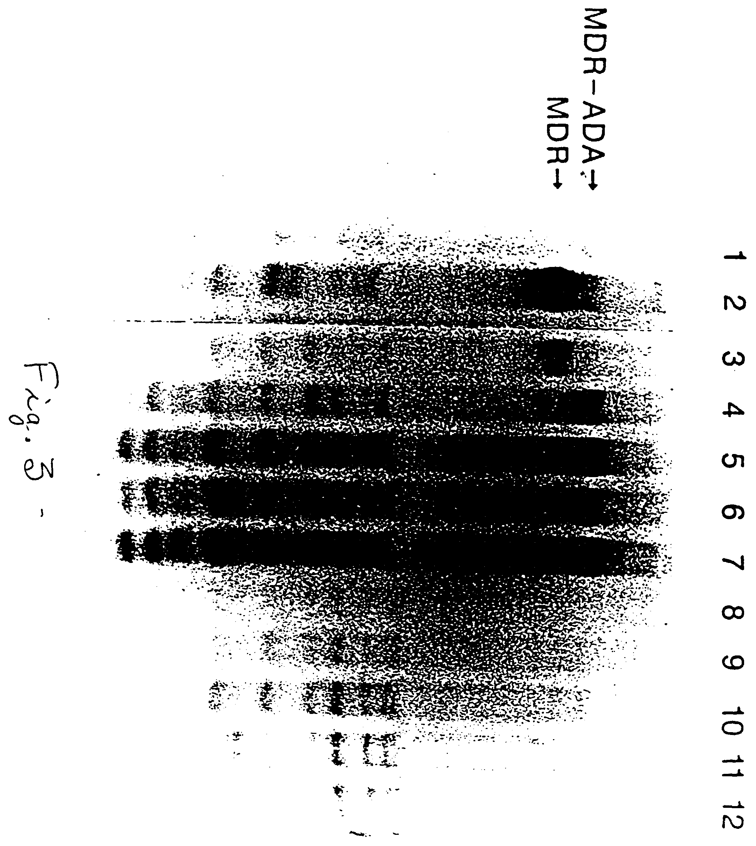

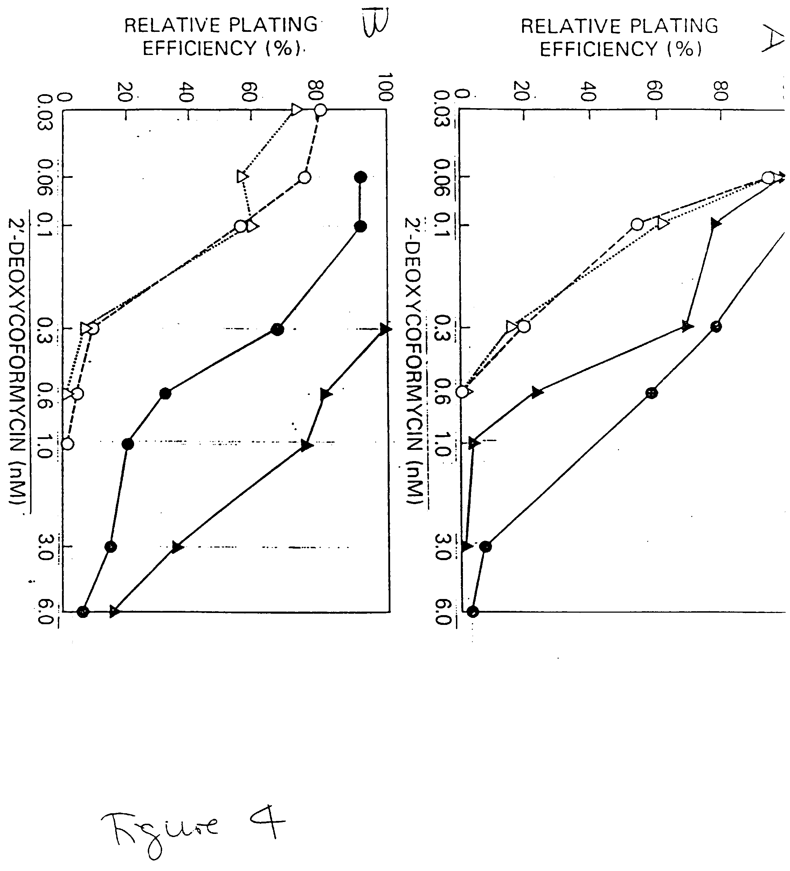

Figure 4 shows killing curves for control and pHaMDR1ADA cell lines. In each experiment 300 cells were plated in a 60 mm dish containing 5 ml culture medium supplemented with 1.1 mM adenosine, 1.0 mM uridine, 0.05 mM alanosine and variable amounts of 2'-deoxycoformycin. After a grown period of ten days at 37°C and 7% CO₂, cells were stained with methylene blue and colonies were counted.
Figure 4A shows individual clones selected at 6 ng/ml colchicine.
   (Δ) KB-A, mock-transfected clones.
   (o) KB-MDR-A, pHaMDR1 transfected clone.
   (▲) KB-MDR1ADA-G, pHaMDR1ADA transfected clone.
   (●) KB-MDR1ADA-I, pHaMDR1ADA transfected clone; and
Figure 4B shows collected cell populations grown at 16 ng/ml (circles) and 24 ng/ml (triangles) colchicine Dashed lines and open symbols (o,Δ) represent pHaMDR1 transfected cells, and solid lines and closed symbols (●,▲) are pHaMDR1ADA transfected cells.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described.

Unless mentioned otherwise, the techniques employed herein are standard methodologies well known to one of ordinary skill in the art.

The term "fusion gene" is defined herein as a DNA segment in which two or more genes are fused resulting in a single open reading frame for coding two or more proteins that as a result of this fusion are joined by one or more peptide bonds.

The concept of "gene therapy" utilizing the gene fusion expression system of the present invention is now exemplified by the construction, transfer and reliably efficient expression of the ADA gene simultaneously with a human MDR1 selectable marker gene, producing a bifunctional chimeric protein in the host cells.

The ADA gene was chosen for the illustrative purposes herein because adenosine deaminase (ADA; adenosine aminohydrolase: E.C. 3.5.4.4) deficiency is a genetic disorder which is associated with approximately one quarter of all cases of severe combined immunodeficiency (Hirschhorn, et al, 1979, Clin. Immunol. Immunopathol, 14:107). This disease is invariably fatal unless effectively treated. ADA catalyzes the irreversible deamination of adenosine and deoxyadenosine to inosine and deoxyinosine, respectively. Most ADA deficient patients produce a catalytically defective enzyme. As a consequence, the cytotoxic ADA substrates, adenosine and deoxyadenosine, as well as their metabolites, particularly deoxyadenosine 5'-triphosphate, are accumulated intracellularly. This leads to the specific destruction of T-lymphocytes and, to a lesser extent, B-lymphocytes, with consequent severe immunological dysfunction.

One of the therapies available for ADA deficiency is bone marrow transplantation from a normal histocompatible donor. However, for many patients, there are no suitable bone marrow donors. Alternative forms of treatment such as enzyme replacement by repeated erythrocyte transfusions or repeated intramuscular injection of polyethylene glycol-modified bovine ADA are also available, but these can lead to severe complications during long-term therapy.

As suggested above, retrovirus-mediated transfer and expression of the ADA gene has been reported in a variety of human T and B lymphocyte cell lines, diploid human skin fibro-blasts, as well as in murine NIH 3T3 and lymphoid cells and, more recently, a Maloney murine leukaemia virus-based recombinant retrovirus has been used to express ADA in murine haematopoietic stem cells (Lim, et al, 1987, Mol. Cell. Biol. 7, 3459). Such retroviral constructs, however, contained no dominant marker gene which would allow selection and thus efficient enrichment of ADA expressing cells.

It is noteworthy that all recombinant retroviral constructs made so far carry, in addition to the ADA gene, a dominant selectable marker gene (e.g., neomycin phosphotransferase, hypoxanthine phosphoribosyl transferase or dihydrofolate reductase) and consistently expressed ADA in vitro, but failed to express ADA in vivo, i.e., in stem cells of experimental animals (Williams, et al, 1986, Proc. Natl. Acad. Sci. USA 83, 2566; McIvor, et al, 1987, Mol. Cell Biol. 7 838; Zwiebel, et al, 1986, Blood 68, 307).

The present invention is the first to directly link the expression of the human MDR1 selectable marker gene to the expression of the ADA gene by creating a fusion MDR1-ADA gene which directs the synthesis of a bifunctional chimeric protein in the recipient cells of the host simultaneously conferring multidrug resistance and efficient ADA activity.

### EXAMPLE 1

### CONSTRUCTION OF A RETROVIRAL VECTOR CONTAINING A MDR1-ADA FUSION GENE

As shown schematically in Fig. 1, a human MDR1 cDNA (see Ueda et al. Proc. Natl. Acad. Sci. (1987), 84, 3004-3008) was fused to a human ADA cDNA by a synthetic linker and placed between the 5' and 3' long terminal repeats (LTRs) of the Harvey murine sarcoma virus expression vector pC06-HX following standard procedures such as described by Velu, et al., 1987, Science 238, 1408. All intermediates as well as the final constructs were characterized by restriction endonuclease mapping.

As a first step (Fig. 1A), a 1.1 kb Asp 718 - HhaI fragment encoding a carboxylterminal region of the MDR1 gene product P-glycoprotein (ranging from Val-926 to Lys-1278) was isolated from plasmid pMDR1 104-2. Two oligonucleotides, a 12-mer and an 18-mer, were synthesized and annealed to give an adapter with HhaI and HindIII compatible ends and containing a single SalI restriction site. The adapter encodes the two carboxyl terminal amino acid residues of P-glycoprotein.

In a second step shown in Fig. 1B full-length human ADA cDNA corresponding to ADA 211 cDNA as described by Adrian, et al., 1984, Hum. Genet. 68, 169, was subcloned as a 1.5 kb EcoRI fragment into the plasmid pUC8. Clone pUC8 ADA was then partially digested with NcoI to yield two types of linearized molecules which were both isolated by agarose gel electrophoresis. The desired linearized form of pUC8 ADA was cleaved within the codon for the initiator-methionine of ADA. In order to restore this codon, the ends of the linearized plasmid were filled in using the Klenow fragment of DNA polymerase I. Then a non-phosphorylated SalI-linker [carrying the new amino acids (Arg-Pro) of the final tripeptide-junction between P-glycoprotein and ADA] was added by ligation and plasmid pESE-13 was obtained.

In the next step outlined in Fig. 1C, the human MDR1 and ADA genes were fused at the newly created single SalI site located at the 3' end of the P-glycoprotein coding region (pAHSH-1) and at the 5' end of the ADA structural gene (pESE-13). Concomitantly, the fusion gene was inserted into the pGEM-2 vector (Promega). To this end a 2.9 kb EcoRI - SacI fragment (containing pGEM-2 plasmid sequences) and a 2.9 kb SacI - Asp 718 fragment encoding the aminoterinal part of P-glycoprotein) were isolated from pMDR1 2000XS (Ueda, et al, 1987, Acad. Sci. USA 84, 3004) and ligated with a 1.1. kb Asp 718 - SalI fragment of pAHSH-1 (carrying the coding region for the carboxylterminal part of P-glycoprotein) and a 1.4 kb SalI - EcoRI fragment isolated from pESE-13 (containing the structural gene for ADA). In the resulting plasmid (pMDRADAS) a single SacII site was present at the 5' end of the fusion gene and could be used for its transfer into the retroviral expression vector pCO6-HX (Velu, et al, 1987, Science 238, 1408).

For this purpose, however, a second site, a single XhoI site, had to be newly created at the 3' end of the fusion gene. Therefore, pMDRADAS was partially cut with EcoRI to linearize the molecule, filled in using the Klenow fragment of DNA polymerase I, and ligated with non-phosphorylated XhoI - linkers. The fusion gene was then excised from pMDRADA XS as a 5.45 kb SacII-XhoI fragment and ligated with the 10.7 kb SacII - XhoI fragment derived from pHaMDR1 and containing the retroviral vector sequences. The final construct is designated pHaMDR1ADA and carries the human MDR1-ADA fusion gene between the 5' and 3' LTRs of Harvey murine sarcoma virus. The MDR1-ADA fusion gene is the only functional eucaryotic gene in this vector and encodes a chimeric protein with an expected Mr of 210 kD. It consists of P-glycoprotein which is connected at the carboxyl-terminal amino acid Gln-1280 to the initiator-methionine of ADA by the tripeptide Gly-Arg-Pro.

A deposit of the pHaMDR1ADA has been made at the American Type Culture Collection (ATCC), 12301 Parklawn Drive, Rockville, Maryland, U.S.A. on May 18, 1988 under the accession number 67699.

### TRANSFECTION OF DRUG-SENSITIVE HUMAN KB CELLS AND COLCHICINE SELECTION

Drug-sensitive human KB-3-1 cells were transfected with pHaMDR1ADA DNA. Negative control cells received no DNA whereas positive control cells were transfected with pHaMDR1 which represents full-length human MDR1 DNA in the same Harvey murine sarcoma virus expression vector (see Fig. 1C). This plasmid represents full-length human MDR1 cDNA in the same Harvey murine sarcoma virus expression vector (see Fig. 10). This plasmid confers the full phenctype of multidrug resistance to a variety of mouse and human cell lines.

Plasmid DNA used for cell transfections was isolated by standard alkaline lysis followed by cesium chloride gradient centrifugation. Drug-sensitive human KB-3-1 cells were transfected by the standard calcium phosphate precipitation method. 10 µg of plasmid DNA were used to transfect 5 x 10⁵ cells per 10 cm dish. Sixteen hours after transfection cells were washed twice and 24 hrs later they were split 1:5 into medium containing the selective drug colchicine at a concentration of 6 ng/ml. After a growth period of ten days, two dishes of cells were stained with 0.5% (w/v) methylene blue in 10% (v/v) ethanol and individual colonies counted. The data presented in Table I show the relative transfection efficiencies of plasmids pHaMDR1ADA and pHaMDR1.

From three non-stained dishes six individual colchicine-resistant colonies were picked and representative cell pools were collected. Both individual clones and cell populations were grown for at least ten more days in the presence of 6 ng/ml colchicine. It has been shown that due to amplification of the endogenous MDR1 gene, human multidrug resistant cell lines become increasingly drug resistant when the concentration of the selective drug in the growth medium is raised. Hence, to investigate whether the transferred MDR1 or MDR1ADA DNA sequences are also amplified, the concentration of colchicine in the culture medium of transfected cells was raised stepwise in two-fold increments up to 96 ng colchicine/ml. Both individual clones and cell populations were passaged twice in appropriate drug concentrations before being plated in the next higher concentration. No difference in growth rate at any concentration of colchicine was observed between pHaMDR1 and pHaMDR1ADA transfected KB cells, indicating that the ADA fusion does not affect the functional activity of P-glycoprotein in the chimeric protein.

### GENOMIC DNA ANALYSIS OF TRANSFECTED KB CELLS

In order to confirm the presence of the introduced MDR1 or MDR1ADA genes and to investigate their copy number, genomic DNA was isolated from transfected and control KB cells by standard procedures (Chen, et al, 1986, Cell 47, 381). Equal amounts of genomic DNA were analyzed by restriction endonuclease digestion using EcoRI, followed by agarose gel electrophoresis, Southern transfer and hybridization to a MDR1-specific probe. A 3 kb fragment of pHaMDR1 transfected cells and a 4 kb fragment of pHaMDR1ADA transfected cells were expected to give rise to a hybridization signal. Indeed, as shown in Fig. 2, strong hybridization signals of the correct size were obtained both for transfected individual clones and cell populations indicating that the integrated DNA sequences were intact. A weak 1.8 kb hybridization signal was detected in all investigated cells including the parental KB-3-1 cell line a a mock-transfected KB clone. Without being bound to any theory, it is postulated that this signal is probably derived from the endogenous single-copy MDR1 gene. The intensity of the 3 kb and 4 kb hybridization signals indicate that the pHaMDR1 and PHaMDR1ADA transfected cells contain multiple copies of the introduced DNA sequences. However, from the data presented in Fig. 3, it cannot be definitively concluded whether increasing concentrations of colchicine in the culture medium caused further amplification in the cell populations (Fig. 2B, lanes 2 and 4) could simply be a reflection of an enrichment for cells which express high levels of the MDR1 or MDR1ADA gene even at low concentrations of colchicine. Moreover, a potential rearrangement of the introduced DNA sequences in pHaMDR1ADA transfected cells (Fig. 2B, lane 4) is also possible.

### ANALYSIS OF THE PROTEINS PRODUCED BY TRANSFECTED KB CELLS

Having confirmed the presence of the introducea DNA sequences in pHaMDR1ADA and pHaMDR1 transfected KB cells, it was important to investigate their expression. To this end immunoprecipitations using a polyclonal rabbit antiserum against the carboxyl terminal regions of P-glycoprotein were performed. Cell cultures were radiolabeled with [³⁵S]-methionine for 16 hours and cell lysates prepared. Antigen-antibody complexes were allowed to form for 18 hours at 4°C and were then precipitated with Protein A Sepharose (SEPHAROSE is a Registered Trade Mark). The precipitated proteins were analyzed by electrophoresis on a SDS - 7% polyacrylamide gel followed by fluorography. As shown in Fig. 3, P-glycoprotein with a Mr of 170 kD was detected in pHaMDR1 transfected cells. The immunoprecipitated protein from pHaMDR1ADA transfected cells has a higher Mr which is in good agreement with the calculated Mr of 210 kD of the MDR1ADA fusion protein. Furthermore, the levels of the chimeric protein seem to increase along with the colchicine resistance of the cells.

### ANALYSIS OF ADA ACTIVITY IN TRANSFECTED KB CELLS

After the expressed MDR1ADA fusion protein was demonstrated to be intact, it was essential to test whether the ADA part was functionally active. To demonstrate ADA function, the sensitivity of pHaMDR1ADA transfected cells to 2'-deoxycoformycin (dCF) was investigated in the presence of toxic concentrations of adenosine and compared to the sensitivity of pHaMDR1 transfected cells. dCF is a tight-binding transition-state analog inhibitor of ADA (Dd = 2.5 x 10⁻¹², Agarwal, et al, 1977, Biochem. Pharmacol. 26, 359; Frieden, et al, 1980, Biochemistry 19, 5303). Under conditions where ADA activity is required (such as in the presence of cytotoxic amounts of adenosine) dCF can be used to estimate intracellular ADA levels. The amounts of dCF necessary to inhibit cell growth correlated with the amounts of functional ADA. Killing curves were performed by growing a constant number of clels (initially 300 cells were plated in a 60 mm dish) in culture medium supplemented with 1.1 mM adenosine, 1.0 mM uridine, 0.05 mM alanosine and variable amounts of dCF. Alanosine was added to block de novo AMP synthesis and uridine to alleviate the block in UMP synthesis caused by the high adenosine concentration. After incubation at 37°C in 7% CO₂ for 10 days, the cells were stained with 0.5% (w/v) methylene blue in 50% (v/v) ethanol and colonies were counted. The results are presented in Fig. 4 and ID₅₀ values are shown in Table II. The ID₅₀ value corresponds to the concentration of dCF which reduces plating efficiency to 50% of the control without dCF. The data clearly indicate that pHaMDR1ADA transfected cells survive higher concentrations of dCF than pHaMDR1 transfected cells or mock-transfected control cells. Hence, it is concluded that ADA, as part of the chimeric MDR1-ADA fusion protein, is functional. Evidence for enzymatic activity, located primarily in the membrane fraction of transfected cells, is shown in Table III.

It should be noted that MDR1 is only an example of a selectable marker gene. Given the illustrative methodology described herein, of course any selectable marker gene can be similarly employed to produce a chimeric gene. Furthermore, the linked gene which is introduced by fusion with the selectable marker gene may be altered (for example by mutation) and the effect of such alternations determined in intact living cells. Such manipulations allow the determination of the effect of the alternation and the verification that the mutant gene is in fact introduced and expressed as a polypeptide. In the current example, the human MDR1 gene linked to the ADA gene could be used to introduce mutant ADA proteins into cells for determining the function of different parts of the ADA molecule. Since ADA is also a selectable marker gene in tissue culture cells via the deoxycoformycin selection, ADA can be used as the selectable marker to introduce an altered MDR1 gene into cells thereby allowing the determination of the function of various modifications of the MDR1 protein.

**TABLE I:**

| Transfections of human KB-3-1 cells with pHaMDR1ADA and pHaMDR1 | |
|---|---|
| DNA (amount) | Colonies/5 x 10⁵ cells |
| pHaMDR1ADA (5 µg) | 75 |
| pHaMDR1ADA (10 µg) | 125 |
| pHaMDR1 (5 µg) | 330 |
| pHaMDR1 (10 µg) | 550 |
| no DNA | 5 |

KB-3-1 cells were plated at 5 x 10⁵ cells/10 cm dish and transfected the next day with the indicated DNAs. After 2 days cells were trypsinized and split 1:5 into culture medium supplemented with 6 ng/ml colchicine. Ten days later colonies were stained with methylene blue and counted.

**TABLE II:**

| Sensitivity of transfected KB cell lines to 2'-deoxycoformycin in the presence of toxic concentrations of adenosine | | | |
|---|---|---|---|
| Cell Line | DNA Used for Transfection | Colchicine Concentration of Selection (ng/ml) | ID₅₀ Value (nM dCF) |
| KB-A | None | 6 | 0.13 |
| KB-MDR1-A | pHaMDR1 | 6 | 0.11 |
| KB-MDR1ADA-G | pHaMDR1ADA | 6 | 0.40 |
| KB-MDR1ADA-I | pHaMDR1ADA | 6 | 0.80 |
| KB-MDR1 Pool | pHaMDR1ADA | 6 | 0.13 |
| KB-MDR1 Pool | pHaMDR1 | 24 | 0.11 |
| KB-MDR1ADA Pool | pHaMDR1ADA | 6 | 0.42 |
| KB-MDR1ADA Pool | pHaMDR1ADA | 24 | 2.0 |

KB-A, KB-MDR1, KB-MDR1ADA G, and KB-MDR1ADA I are individual clones.

**TABLE III**

| ADA ACTIVITY OF CRUDE MEMBRANE AND CYTOSOLIC FRACTIONS OF PARENTAL AND TRANSFECTED KB CELL LINES | | | | |
|---|---|---|---|---|
| Cell line | DNA Used for Transfection | Colchicine Concentration for Selection (ng/ml) | ADA Activity (nmol Inosine/min/mg) | |
| | | | Cytosol | Membranes |
| KB-3-1 | None | 0 | 18.2 | 3.0 |
| KB-MDR1 Pool | pHaMDR1 | 6 | 13.6 | 2.0 |
| KB-MDR1 Pool | pHaMDR1 | 48 | 15.6 | 1.8 |
| KB-MDR1ADA Pool | pHaMDR1ADA | 6 | 15.8 | 1.9 |
| KB-MDR1ADA Pool | pHaMDR1ADA | 24 | 13.3 | 94.9 |
| KB-MDR1ADA Pool | pHaMDR1ADA | 48 | 17.1 | 153.4 |

ADA assays were performed according to Yeung, C.-y., Ingolia, D. E., Bobonis, C., Dunbar, B. S., Riser, M. E., Siciliano, M. J., and Kellems, R. E. (1983) J. Biol. Chem. 258, 8338-8345. To determine ADA activity in the membrane or cytosolic fraction, cells were collected by scraping into PBS, washed twice with PBS, resuspended in hypotonic lysis buffer (10 mM Tris HC1 pH 7.5, 10 mM NaCl, 1 mM MgCl₂) at a concentration of 2 x 10⁷ cells/ml and incubated in an ice-bath for 15 min. The swollen cells were disrupted with 20 strokes in a tightly fitting Dounce homogenizer and the nuclei removed by centrifugation at 400 xg for 10 min at 4°C. The pellet obtained by subsequent centrifugation at 30,000 xg for 30 min at 4°C was used as the crude membrane fraction and the supernatant was used as the cytosolic fraction.

## Claims

1. A fusion gene capable of being expressed in vivo and comprising a coding sequence for a selectable marker linked by fusion to a second coding sequence, resulting in a single open reading frame encoding a chimeric fusion protein, which it is desired to express in recipient cells, characterised in that the selectable marker is a human MDR1 gene.

2. A fusion gene as claimed in claim 1, wherein said second coding sequence is an ADA gene.

3. A fusion gene as claimed in claims 1 or 2, wherein said fusion gene has the characteristics of the MDR1ADA fusion gene carried in plasmid ATCC 67699.

4. A fusion gene as claimed in claim 3, wherein said fusion gene is cloned in an expression vector.

5. A fusion gene as claimed in claim 4, wherein the resultant expression vector has the identifying characteristics of ATCC 67699.

6. A method of determining the effect of altering the sequence of a fusion gene as claimed in claim 1, comprising the step of altering the second coding sequence and determining the resultant effect of said alteration on the function of said fusion gene.

7. A fusion gene, as claimed in any of claims 1-5, for use in a method of alleviating a condition resulting from a genetic or an acquired disorder related to the second coding sequence, wherein the fusion gene is introduced into cells of a host and the second coding sequence is expressed in vivo.

8. Use of a fusion gene, as claimed in any one of claims 1-5, for the manufacture of a medicament for use in a method for alleviating a condition resulting from a genetic or acquired disorder related to the second coding sequence, wherein the fusion gene is introduced into cells of a host and the second coding sequence is expressed in vivo.

9. A bifunctional chimeric protein encoded by a fusion gene according to any one of claims 1-5 or 7.

10. A bifunctional chimeric protein as claimed in claim 9, comprising a human MDR1 gene product, P-glycoprotein, which is linked at its carboxy-terminal amino acid residue Gln-1280 to the amino-terminal amino acid of a gene product of a second coding sequence.

11. A bifunctional chimeric protein as claimed in claim 10, wherein the carboxy-terminal amino acid residue Gln-1280 of the P-glycoprotein is linked to the amino terminal amino acid of the gene product of the second coding sequence by a short peptide having the sequence Gly-Arg-Pro.

12. A bifunctional chimeric protein as claimed in claim 11, wherein the short peptide is connected to the initiator methionine of ADA protein.

## Patentansprüche

1. Fusioniertes Gen, das im lebenden Organismus exprimierbar ist und eine kodierende Sequenz für einen wählbaren Marker aufweist, der durch Verschmelzung an eine zweite kodierende Sequenz gekoppelt ist, was ein einzelnes offenes Leseraster ergibt, das ein chimerisches Fusionsprotein kodiert, das in Empfängerzellen exprimiert werden soll, dadurch gekennzeichnet, daß der wählbare Marker ein menschliches MDR1-Gen ist.

2. Fusioniertes Gen nach Anspruch 1, wobei die zweite kodierende Sequenz ein ADA-Gen ist.

3. Fusioniertes Gen nach Anspruch 1 oder 2, wobei das fusionierte Gen die Eigenschaften des im Plasmid ATCC 67699 geführten MDR1ADA-Fusionsgens aufweist.

4. Fusioniertes Gen nach Anspruch 3, wobei das fusionierte Gen in einem Expressionsvektor kloniert ist.

5. Fusioniertes Gen nach Anspruch 4, wobei der resultierende Expressionsvektor die identifizierenden Eigenschaften von ATCC 67699 aufweist.

6. Verfahren zum Bestimmen der Auswirkung einer Änderung der Sequenz eines fusionierten Gens nach Anspruch 1, welches den Schritt der Änderung der zweiten kodierenden Sequenz und des Bestimmens der resultierenden Auswirkung dieser Änderung auf die Funktion des genannten fusionierten Gens umfaßt.

7. Fusioniertes Gen nach einem der Ansprüche 1 bis 5 zur Verwendung in einem Verfahren zur Linderung eines Zustands, der von einer genetischen oder einer erworbenen Krankheit herrührt, die mit der zweiten kodierenden Sequenz in Zusammenhang steht, wobei das fusionierte Gen in Zellen eines Wirts eingeführt werden und die zweite kodierende Sequenz im lebenden Organismus exprimiert wird.

8. Verwendung eines fusionierten Gens nach einem der Ansprüche 1 bis 5 zur Herstellung eines Medikaments zur Verwendung bei einem Verfahren zur Linderung eines Zustands, der von einer genetischen oder erworbenen Krankheit herrührt, die mit der zweiten kodierenden Sequenz im Zusammenhang steht wobei das fusionierte Gen in Zellen eines Wirts eingeführt wird und die zweite kodierende Sequenz im lebenden Organismus exprimiert wird.

9. Bifunktionales chimerisches Protein, das durch ein fusioniertes Gen nach einem der Ansprüche 1 bis 5 oder 7 kodiert ist.

10. Bifunktionales chimerisches Protein nach Anspruch 9, das ein menschliches MDR1-Genprodukt, P-Glycoprotein, aufweist, das an seinem karboxy-terminalen Aminosäurerest Gln-1280 an die amino-terminale Aminosäure eines Genprodukts einer zweiten kodierenden Sequenz gekoppelt ist.

11. Bifunktionales chimerisches Protein nach Anspruch 10, wobei der karboxy-terminale Aminosäurerest Gln-1280 des P-Glycoproteins an die aminoterminale Aminosäure des Genprodukts der zweiten kodierenden Sequenz durch ein kurzes Peptid mit der Sequenz Gly-Arg-Pro gekoppelt ist.

12. Bifunktionales chimerisches Protein nach Anspruch 11, wobei das kurze Peptid mit dem Initiator-Metionin von ADA-Protein verbunden ist.

## Revendications

1. Gène de fusion susceptible d'être exprimé in vivo et comprenant une séquence codante pour un marqueur identifiable lié par fusion à une seconde séquence codante, donnant un seul cadre de lecture ouvert codant pour une protéine de fusion chimérique, dont on recherche l'expression dans les cellules receveuses, caractérisé en ce que le marqueur identifiable est un gène MDR1 humain.

2. Gène de fusion selon la revendication 1, dans lequel ladite seconde séquence codante est un gène de l'ADA.

3. Gène de fusion selon la revendication 1 ou 2, dans lequel ledit gène de fusion présente les caractéristiques du gène de fusion MDR1ADA présent dans le plasmide ATCC 67699.

4. Gène de fusion selon la revendication 3, dans lequel ledit gène de fusion est clone dans un vecteur d'expression.

5. Gène de fusion selon la revendication 4, dans lequel le vecteur d'expression résultant présente les caractéristiques d'identité du plasmide ATCC 67699.

6. Procédé de détermination de l'effet d'une altération de la séquence d'un gène de fusion selon la revendication 1, comprenant l'étape d'altération de la seconde séquence codante et de détermination de l'effet résultant de ladite altération sur la fonction dudit gène de fusion.

7. Gène de fusion selon l'une quelconque des revendications 1 à 5, destiné à être utilisé dans un procédé visant à traiter une pathologie résultant d'un trouble génétique ou acquis lié à la seconde séquence codante, procédé selon lequel le gène de fusion est introduit dans des cellules d'un hôte et la seconde séquence codante est exprimée in vivo.

8. Utilisation d'un gène de fusion tel que revendiqué selon l'une quelconque des revendications 1 à 5, pour l'obtention d'un médicament destiné à être utilisé dans un procédé visant à traiter une pathologie résultant d'un trouble génétique ou acquis lié à la seconde séquence codante, procédé selon lequel le gène de fusion est introduit dans des cellules d'un hôte et la seconde séquence codante est exprimée in vivo.

9. Protéine chimérique bifonctionnelle codée par un gène de fusion selon l'une quelconque des revendications 1 à 5 ou 7.

10. Protéine chimérique bifonctionnelle selon la revendication 9, comprenant un produit du gène MDR1 humain, la glycoprotéine P, qui est liée par le résidu de l'acide aminé Gln-1280 carboxy-terminal à l'acide aminé amino-terminal d'un produit génique d'une seconde séquence codante.

11. Protéine chimérique bifonctionnelle selon la revendication 10, dans laquelle le résidu de l'acide aminé Gln-1280 carboxy-terminal de la glycoprotéine P est lié à l'acide aminé amino-terminal du produit génique de la seconde séquence codante par un peptide court dont la séquence est Gly-Arg-Pro.

12. Protéine chimérique bifonctionnelle selon la revendication 11, dans laquelle le peptide court est lié à l'initiateur méthionine de la protéine ADA.
